# EUROPÄISCHE PATENTSCHRIFT

(11) **EP 2 219 562 B1**
(45) Veröffentlichungstag und Bekanntmachung des Hinweises auf die Patenterteilung: **10.02.2016**
(21) Anmeldenummer: 08853677.6
(22) Anmeldetag: 27.11.2008
(51) Int. Cl.: A61F 2/44

(54) **ZWISCHENWIRBELIMPLANTAT**
INTERVERTEBRAL IMPLANT
IMPLANT INTERVERTÉBRAL

(30) Priorität: 27.11.2007 DE 102007056993
(43) Veröffentlichungstag der Anmeldung: 25.08.2010
(73) Patentinhaber: Kloss, Henning, 6373 Ennetbürgen (CH)
(72) Erfinder: KRAUS, Kilian, 97440 Werneck (DE)
(74) Vertreter: Arth, Hans-Lothar
(86) Internationale Anmeldenummer: PCT/DE2008/001994
(87) Internationale Veröffentlichungsnummer: WO 2009/068021

(56) Entgegenhaltungen:
- EP-A- 0 621 018
- EP-A- 0 719 529
- WO-A-96/40014
- WO-A-2006/053291
- US-A- 5 732 469
- US-A1- 2005 112 397

## Beschreibung

Die vorliegende Erfindung betrifft Zwischenwirbelimplantate, sogenannte Cages, mit einer inneren kanalartigen Struktur.

Im Stand der Technik sind massive als auch hohle Implantate insbesondere für den Wirbelsäulenbereich bekannt, welche entweder aufgrund ihrer massiven Struktur das Einwachsen von Knochenzellen nicht zulassen oder einen zu großen Hohlraum bieten, der nicht vollständig mit körpereigenen Knochenzellen innerhalb vertretbarer Zeit ausgefüllt werden kann und daher in der Regel mit Knochenersatzmaterial oder Knochensplittern künstlich befüllt wird.

Ziel einer Fusion beispielsweise mittels Cages im Wirbelsäulenbereich ist die knöcherne Durchbauung, da man eine möglichst langfristige Stabilität erreichen möchte. Durchbaut der Knochen das Implantat so erreicht man den Vorteil, dass sich die Knochenzellen wie sonst im Körper auch, erneuern können und somit die Langfristigkeit gewährleistet ist. Die Cages dienen somit als vorübergehender Platzhalter, damit das Bandscheibenfach nicht einsinkt und somit an Höhe verliert. Deshalb müssen die Cages primär auch die Statik übernehmen, zumindest solange bis die Durchbauung des Implantats stattgefunden hat. Eine zügige und stabile knöcherne Durchbauung eines künstlichen Bandscheibenimplantates wie z.B. eines Cages möchte man grundsätzlich erreichen, da derartige Implantate der natürlichen Bandscheine am nächsten kommen und für den Patienten die vorteilhafteste Ausführungsform darstellen.

Der Nachteil eines massiven Implantats wie z.B. eines massiven Cages liegt offensichtlicher Weise darin, dass gar keine Durchbauung des Implantats möglich ist, d.h. das Implantat dauerhaft die Stützfunktion übernehmen muss und somit langfristig weniger wirksam ist. Dient ein Implantat als reiner Abstandshalten, so besteht des weiteren die Gefahr, dass das Implantat in den Knochen einsinkt und der gewünschte Abstand nicht mehr gewährleistet ist. Derartige Nachteile könnten z.B. dadurch vermieden werden, dass das Implantat vom Knochen natürlich durchbau wird.

Als eine Stand der Technik Ausführungsform offenbart EP 0 621 018 A1 in den Figuren 26 und 27 sowie auf Seite 12 in Beispiel 3 ein Zwischenwirbelimplantat AL mit einem massiven künstlichen Zentralköroer AL1, der beispielsweise aus einer Titanlegierung bestehen kann. Die Flächen des Zentralkörpers AL1, welche den angrenzenden Wirbeln zugewandt sind, wurden jeweils laminiert mit einem formpassenden Bauteil AL2, welches aus miteinander laminierten und unterschiedlich ausgeschnittenen Platten besteht, so dass sich auf der Oberseite aus auch der Unterseite des Zwischenwirbelimplantats AL eine nicht massive Struktur ergibt, in welche neu gebildeter Knochen einwachsen kann. Aufgrund der aufeinander laminierten und unterschiedlich ausgesparten Platten ergibt sich ein Kanalnetzwerk aus vertikal verlaufenden miteinander verbundenen Kanälen, Die aufeinander laminierten ca. 10 Platten bestehen vorzugsweise aus Titan haben jeweils eine Dicke von 100 µm, so dass sich Bauteile AL2 von ca. 1 mm Dicke ergeben. Diese Kanäle verlaufen zwar durch die Bauteile AL2 jedoch nicht durch das gesamte Zwischenwirbelimplantat AL von der Oberseite zur Unterseite, weil der Zentralkörper AL1 massiv ist.

Hohle Implantate wie z.B. hole Cages kommen mit oder ohne Knochenersatzstoffe zur Anwendung. Diese Implantate haben jedoch wiederum den Nachteil, dass der Knochen einen zu großen Hohlraum durchbauen müsste, falls keine Knochenersatzstoffe verwendet werden, um die Implantate zu füllen und daher das Implantat wiederum zu lange die Stützfunktion mit den oben geschilderten Nachteilen übernehmen müsste. Werden Knochenersatzstoffe verwendet, so dienen diese dazu, den Knochenbau anzuregen. Da Blut den Katalysator für die Knochenbildung darstellt, der mit Knochenersatzstoff gefüllte innere Hohlraum des Cages jedoch nicht ausreichend mit Blut versorgt wird, findet ein natürliches Knochenwachstum zur Durchbauung des teilweise mit Knochenersatzstoff gefüllten Cages nur ungenügend statt. Dies hat wiederum zur Folge, dass eine Durchbauung eines zum Teil mit Knochenersatzstoff gefüllten Cages auch nicht in gewünschter Weise stattfindet.

Ideal wäre daher eine bioresorbierbare künstliche Bandscheibe, welche so lange die Stützfunktion übernimmt, bis der körpereigene Knochen diese ersetzt hat und die Stützfunktionen übernehmen kann. Derartige Ausführungsformen sind aufgrund fehlender geeigneter Materialien bisher nicht realisiert worden. Die liegt zum einen daran, dass keine biologisch abbaubaren Materialien zur Verfügung stehen, welche eine ausreichende Stabilität gewährleisten während sich der Knochen aufbaut und ferner sich nicht in der Abbaugeschwindigkeit ausreichend genau regulieren lassen, weil der Aufbau des Knochens und der Abbau des Implantats mit exakt gleicher Geschwindigkeit erfolgen müssen, damit keine Übergangsstruktur entsteht, welche kollabieren könnte.

Wünschenswert wären hingegen knochenverbindende oder knochenüberbrückende Implantate, welche zum einen eine ausreichende mechanische Stabilität bieten und zudem möglichst vollständig mit körpereigenem Knochen durchbaut bzw. durchwachsen werden können.

Aufgabe der vorliegenden Erfindung ist somit die Bereitstellung von Zwischenwirbelimplantaten, welche das Einwachsen von körpereigenem Knochen bestmöglich fördern, um dadurch den körpereigenen Knochenaufbau bestmöglichst zu unterstützen und bis zum Aufbau des körpereigenen Knochens ausreichende Stabilität gewährleisten.

Diese Aufgabe wird durch die Bereitstellung eines Implantats gemäß Patentanspruch 1 gelöst. Weitere vorteilhafte Ausgestaltungen, Aspekte und Details der Erfindung ergeben sich aus den abhängigen Ansprüchen, der Beschreibung, den Beispielen und den Figuren.

Die vorliegende Erfindung betrifft metallische knochenverbindende oder knochenüberbrückende Zwischenwirbelimplantate in Form von künstlichen Bandscheiben, wobei die künstlichen Bandscheibenimplantate zumindest eine den Knochen kontaktierende Oberfläche und eine innere Struktur aus einer Vielzahl an Kanälen mit definierten Querschnittsflächen oder Radien aufweisen und die Kanäle zudem über Öffnungen miteinander verbunden sind, so dass ein dreidimensionales Kanalnetz entsteht.

Es wurde überraschend gefunden, dass knochenverbindende oder knochenüberbrückende Bandscheibenimplantate besonders gut mit dem kontaktierten Knochen verwachsen, wenn die Oberfläche des Implantats nicht glatt oder nicht rau oder nicht porös ist, sondern eine Kanalstruktur aufweist, wobei die Kanäle untereinander durch Öffnungen verbunden sind und eine definierte Struktur besitzen. Die Art als auch die Symmetrie der Kanalstruktur ist erfindungswesentlich und wird weiter unten ausführlich beschrieben.

Daher betrifft die vorliegende Erfindung ein Zwischenwirbelimplantat, wobei das Implantat zwei Oberflächen zur Kontaktierung von zwei Wirbelkörpern, einen äußeren Mantel und eine innere Struktur aufweist und wobei die innere Struktur aus einer Vielzahl von Kanälen gebildet wird und die Kanäle jeweils eine Querschnittsfläche von 8.000 µm² bis 7.000.000 µm² besitzen und die Kanäle parallel zueinander entlang der Längsachse der Wirbelsäule verlaufen und die Kanäle über Öffnungen miteinander verbunden sind, dadurch gekennzeichnet, dass jeder Kanal durch mindestens zwei Öffnungen mit benachbarten Kanälen verbunden ist und sich die Öffnungen durchgehend von einer knochenkontaktierenden Oberfläche zur gegenüberliegenden Oberfläche in Form von Schnitten erstrecken.

Unter dem Begriff "knochenverbindend" oder "knochenüberbrückend" wird verstanden, dass das Implantat direkt mit einem Knochen in Verbindung steht also zumindest ein Teil der Oberfläche des Bandscheibenimplantats mit einem Knochen in Berührung kommt.

Als Beispiele solcher Bandscheibenimplantate oder Wirbelzwischenimplantate sind insbesondere Cages für die cervicale-, thorakale- oder lumbale Anwendung (wie bspw. ALIF-Cages, PLIF-Cages und TLIF-Cages) zu nennen. Die erfindungsgemäßen Wirbelzwischenimplantate werden auch als Zwischenwirbelkörperelement oder Implantate zur intersomatischen Fusion oder als Implantate zur interkorporellen Wirbelkörperfusion bezeichnet.

Die vorgenannten Implantate sind in der Regel vollständig aus einem harten Material, insbesondere einem Metall oder einer Metalllegierung wie beispielsweise Titan, Zirkonium, oxidiertem Zirkonium, Hafnium, Platin, Rhodium, Niobium, medizinischem Edelstahl, CoCr-Stahl (Cobalt-Crom), Tantal und können aber auch aus faserverstärkten Kunststoffen (Glas- / Carbonfasem mit entsprechender Matrix), PEEK [Poly(etheretherketone)] oder Polymerwerkstoffen allgemein bestehen. Bei den Metalllegierungen können ferner Metalle wie Aluminium, medizinischer Stahl und/oder Gold zugesetzt werden.

Einteilige Bandscheibenimplantate wie die erfindungsgemäßen Cages, welche auch als Wirbelzwischenimplantat bezeichnet werden, weisen in der Regel einen massiven Außenmantel ohne Kanalstruktur auf, um eine ausreichende Stabilität des Implantates zu gewährleisten. Der Begriff "massiv" wie hierin verwendet bedeutet, dass der Außenmantel gar keine Öffnungen also auch keine Öffnungen von Kanälen der erfindungsgemäßen Kanalstruktur oder Öffnungen von Verbindungskanälen zwischen den Kanälen der erfindungsgemäßen Kanalstruktur aufweist bis hin zu einer solchen Anzahl an Öffnungen von Kanälen oder Verbindungskanälen der erfindungsgemäßen Kanalstruktur, dass der Außenmantel bei dem 5-fachen bevorzugt bei dem 8-fachen und insbesondere bevorzugt bei dem 10-fachen durch die Wirbelsäule ausgeübten Druck in Richtung der Längsachse der Wirbelsäule nicht deformiert wird, d.h. die Öffnungen nicht zusammengedrückt werden.

Die erfindungsgemäße Kanalstruktur im Inneren des Cages bzw. des künstlichen Bandscheibenimplantats dient zur gezielten Anregung des Knochenwachstums und weniger zur Stabilisierung des gesamten Implantats. Die mechanische Stabilität wird dem Wirbelzwischenimplantat, dem Cage, durch einen Außenmantel verliehen, welcher zum einen ausgestaltet ist, um den hohen Drücken der Wirbelsäule Stand zu halten und das Einsinken des Implantats in den Wirbelknochen verhindert, so dass der durch die Höhe des Außenmantels definierter Abstand zwischen zwei Wirbelkörper aufrecht erhalten werden kann. Somit ist der Außenmantel, d.h. der Teil des Bandscheibenimplantats, der die innere Kanalstruktur umgibt, massiv und nicht Teil der inneren Kanalstruktur. Der Begriff "massiv" soll so verstanden werden, dass dieser Außenmantel vorzugsweise nicht von Kanälen durchzogen ist, sich darin keine Löcher befinden und keine Kanäle durch den Außenmantel verlaufen und auf dessen Außenfläche enden. Somit kann vorzugsweise auch kein Blutfluß durch den Außenmantel hindurch stattfinden. Der Außenmantel verliert mit der Zeit seine Stützfunktion je mehr die innere Kanalstruktur mit Knochen durchwachsen wird.

Der Außenmantel kann auch als corticale Außenwand des Zwischenwirbelimplantates bezeichnet werden. Die Zwischenwirbelimplantate, die zur intersomatischen Fusion eingesetzt werden, sollten idealerweise der Grundfläche der angrenzenden Wirbelkörper entsprechen.

Der für die knöcherne Durchbauung zur Verfügung stehende Raum soll zudem maximiert werden aber dennoch von körpereigenen Knochenzellen zügig durchwachsen werden können. Gleichzeitig muss im ersten Moment der Versorgung, d.h. nach der Implantation das Implantat die Statik übernehmen und es muss verhindert werden, dass das Implantat dem Wirbelkörper zu wenig Unterstützungsfläche bietet und es deshalb unter Lasteinwirkung in den Wirbelkörper einsinkt.

Die insbesondere lasttragende Struktur des Wirbelkörpers ist die zirkulär umlaufende Corticalis. Im Idealfall kommt die massive Wandung des Cages zwischen den zirkulär verlaufenden Corticaliswänden der angrenzenden Wirbelkörper zu liegen, sodass der Corticalis eine Unterstützungsfläche zur Verfügung steht, die ein Einsinken des Cages in den Wirbelkörper verhindert.

Im Bereich der Spongiosa, d.h. dem gut durchbluteten Knochen zentral im Wirbelkörper gelegen, liegt die wabenartige Struktur des Cages, um eine ideale knöcherne Durchbauung zu gewährleisten.

Die erfindungsgemäßen Implantate können nach Standardtechniken hergestellt werden, beispielsweise unter Verwendung von Lasertechnologie und Laserschneidverfahren sowie Laserschmelzverfahren, z.B. Lasercusing und demnach im Rahmen der beschriebenen Erfindung beliebige Gestalt annehmen.

Die erfindungsgemäßen Cages sind somit vorzugsweise einteilig, bestehen vollständig oder zumindest zu 90 Gew.-% aus Metall oder einer Metalllegierung, sind nicht porös wie beispielweise Keramiken es sein können, sondern weisen eine definierte innere Kanalstruktur auf, welche den Blutfluß unterstützt und damit die besten Vorraussetzungen für körpereigenes Knochenwachstum schafft und weisen einen äußeren Mantel auf, der für die Stabilitätsgebung zumindest so lange verantwortlich ist, solang der neugebildete Knochen diese Funktion noch nicht übernehmen kann.

Der Begriff "einteilige Bandscheibenimplantate" oder "einteilige Cages" bezieht sich nur auf das Implantat selbst und nicht auch auf irgendwelche Befestigungsmittel. Derartige Bandscheibenimplantate können beispielweise in die angrenzenden Wirbelkörper verschraubt werden. Die verwendeten Befestigungsmittel wie z.B. Schrauben werden bei dem Begriff "einteilig" nicht berücksichtigt und werden als Zubehör zu dem erfindungsgemäßen Bandscheibenimplantat bezeichnet wie auch das Implantationswerkzeug. Ferner sind natürliche Materialien wie beispielsweise natürliches Knochenmaterial bei dem erfindungsgemäßen Bandscheibenimplantat kein Bestandteil und bei dessen Implantation muss oder wird auch kein künstlichen Knochenmaterial verwendet. Die erfindungsgemäßen Cages sind somit gemäß dieser Definition vorzugsweise einteilig. Zweiteilige Ausführungsformen sind auch noch möglich, wobei die erfindungsgemäßen Implantate aus maximal drei Teilen vorzugsweise sogar aus nicht mehr als zwei Teilen bestehen, wobei die weiteren Teile in der Regel am Cage vorgesehene Befestigungsmittel betreffen wie beispielsweise entfernbare Platten für Befestigungsschrauben oder Befestigungshaken oder Befestigungskrallen oder dergleichen, welche in der Regel optional an den erfindungsgemäßen Implantaten vorgesehen werden können.

Die erfindungsgemäßen Implantate sind nicht nach einem Baukastenprinzip oder aus mehreren Einzelteilen oder Teilstücken zusammengesetzt, welche sich eventuell nur schwer miteinander verbinden lassen oder gegeneinander translationsbeweglich, rotationsbeweglich oder gleitend verschieben können und besitzen einen Außenmantel mit definierter Form, der nach der Implantation seine Form und Maße nicht mehr verändert.

Eine Möglichkeit besteht jedoch darin, die innere Wabenstruktur oder Kanalstruktur separat von dem Außenmantel zu fertigen und nach getrennter Fertigung zusammen zu fügen, so dass letztendlich auch wieder ein einteiliges Implantat vorliegt. Wie oben beschrieben ist der Außenmantel massiv, d.h. es dürfen maximal so viele Aussparungen, Löcher oder Öffnungen darin enthalten sein, dass eine Deformation durch den Wirbelsäulendruck bis zum Zeitpunkt der vollständigen Durchbauung mit körpereigenem Knochen nicht stattfinden kann. Bevorzugt ist, dass der massive Außenmantel keinerlei Aussparungen, Löcher oder Öffnungen aufweist.

Die erfindungsgemäße Kanalstruktur beginnt an der knochenkontaktierenden Fläche des Implantats, so dass die Öffnungen der Kanäle dem Knochen zugewandt sind, d.h. die oberen Öffnungen sind dem oberen kontaktierten Wirbelkörper zugewandt und die unteren Öffnungen der Kanäle dem unteren Wirbelkörper.

Bei knochenverbindenden oder knochenüberbrückenden Implantaten des Wirbelsäulenbereichs wie auch bei den erfindungsgemäßen Implantaten sind die Kontaktflächen der Implantate mit dem jeweiligen Knochen in der Regel eben und die Kanäle erstrecken sich entlang der Wirbelsäulenlängsachse von der Knochenkontaktfläche weg.

Als Kontaktfläche wird die Fläche des Cages verstanden, welche mit dem aufliegenden Wirbelkörper sowie die gegenüberliegende Fläche des Cages, welche mit dem unterliegenden Wirbelkörper in Berührung kommt.

Die Kontaktfläche mit dem Knochen muss jedoch nicht eben ausgestaltet sein, wie es bei den Wirbelzwischenimplantaeten des Standes der Technik der Fall ist, sondern kann auch eine unsymmetrische Form besitzen. Es ist durchaus weiter bevorzugt, wen sich die innere Kanalstruktur geringfügig über den Außenmantel in Richtung des aufliegenden Wirbelkörpers als auch in Richtung des unterliegenden Wirbelkörpers erstreckt wie weiter unter noch ausführlicher ausgeführt wird. Der sich über den Außenmantel hinaus erstreckende Teil der inneren Kanalstruktur sinkt oder drückt sich in den aufllegenden bzw. den unterliegenden Wirbelkörper ein und führt damit zu einer gewollten Verletzung der Oberfläche dieser beiden Wirbelkörper, wodurch das Knochenwachstum aus auch der Blutfluß abermals gesteigert werden.

Somit beginnen die Kanäle an der knochenkontaktierenden Oberfläche des Implantats, wobei die innere Kanalstruktur eine ebene Oberfläche zum aufliegenden Wirbelkörper als auch eine ebene Oberfläche zum unterliegenden Wirbelkörper aufweisen kann. Bevorzugt ist jedoch eine konvexe, d.h. zum Wirbelkörper gerichtete Krümmung der Oberfläche der inneren Kanalstruktur, wobei die kontaktierende Oberfläche zum aufliegenden Wirbelkörper konvex ausgestaltet sein kann und/oder die den unterliegenden Wirbelkörper kontaktierende gegenüberliegende Oberfläche der inneren Kanalstruktur konvex ausgestaltet sein kann. Die konvexe Krümmung der inneren Kanalstruktur hat eine Höhe gemessen am höchsten Punkt der Krümmung von 0,1 mm bis 5 mm.

Erfindungswesentlich ist, dass die einzelnen Kanäle oder zumindest 75% aller Kanäle, bevorzugt zumindest 85% aller Kanäle und insbesondere bevorzugt mindestens 95% aller Kanäle eine Querschnittsfläche im Bereich von 8.000 µm² bis 7.000.000 µm², bevorzugt von 50.000 µm² bis 3.100,000 µm², weiter bevorzugt im Bereich von 100.000 µm² bis 800.000 µm², noch weiter bevorzugt im Bereich von 125.000 µm² bis 650.000 µm² und insbesondere bevorzugt im Bereich von 160.000 µm² bis 570.000 µm² aufweisen.

Der Ausdruck, dass 85% aller Kanäle eine Querschnittsfläche innerhalb der vorgenannten Bereiche aufweisen bedeutet, dass von 100 Kanälen 85 Kanäle eine Querschnittsfläche im vorgenannten Bereich haben und die restlichen 15 Kanäle eine kleine oder größere als auch eine deutlich kleinere oder deutliche größere Querschnittsfläche aufweisen können.

Die Kanäle können eine beliebige Form aufweisen und rund, oval, dreieckig, viereckig, fünfeckig, sechseckig, siebeneckig, achteckig oder beliebig vieleckig ausgestaltet sein. Bevorzugt sind jedoch Ausgestaltungen mit Innenwinkeln größer als 90°, also begonnen mit einem Fünfeck über ein Vieleck bis hin zum Kreis oder einem Oval. Weiter bevorzugt sind fünfeckige, sechseckige, siebeneckige und achteckige Ausgestaltungen und insbesondere sechseckige Kanäle.

Bei runden Kanälen ist die Querschnittsfläche gleich der Kreisfläche und kann einfach gemäß πr² berechnet werden, wobei r der Radius des Kanals ist.

Bezogen auf runde oder annähernd runde Kanalformen ist bevorzugt, wenn die Kanäle oder zumindest 75% aller Kanäle, bevorzugt zumindest 85% aller Kanäle und insbesondere bevorzugt mindestens 95% aller Kanäle einen Durchmesser von 100 - 3000 µm, bevorzugt 250 - 2000 µm, weiter bevorzugt 350 - 1000 µm, noch weiter bevorzugt 400 - 900 µm und insbesondere bevorzugt 450 - 850 µm aufweisen.

Bei mehreckigen Kanalformen wird als Durchmesser der Abstand von zwei gegenüberliegenden parallelen Flächen bei geradzahligen Vielecken (viereckig, sechseckig, achteckig usw.) oder der Abstand eines Eckpunktes zum Mittelpunkt der gegenüberliegenden Geraden bei ungeradzahligen Vielecken (Dreieck, Fünfeck, Siebeneck usw.) bezeichnet.

Die Wandstärke der Kanalwände beträgt 20 µM bis 700 µM, vorzugsweise 30 µM bis 550 µM und weiter bevorzugt 40 µM bis 400 µM. Der Durchmesser der Kanäle beträgt vorzugsweise das 2-fache bis 4-fache der Kanalwandstärke (Kanalwanddicke). Der Außenmantel weist eine Dicke von 500 µM bis 1.500 µM, vorzugsweise von 700 µM bis 1.300 µM und insbesondere bevorzugt von 850 µM bis 1.100 µM auf. Die Dicke des Außenmantels entspricht vorzugsweise dem 1-fachen bis 2-fachen Durchmesser der Kanäle. Die Dicke der Einschnitte oder Verbindungskanäle oder der Durchmesser der Öffnungen beträgt vorzugsweise ein Drittel bis ein Zehntel der Wandstärke der Kanäle.

Kanäle mit vorgenanntem Durchmesser oder vorgenannter Querschnittsfläche erstrecken sich also von der Fläche des Implantats, welche an dem Knochen anliegt, in das Implantatinnere. Bei den vorzugsweise einteiligen erfindungsgemäßen Implantaten mit gegenüberliegenden knochenkontaktierenden Flächen wie den erfindungsgemäßen Cages erstrecken sich vorzugsweise die Kanäle durch das Implantat bis zur gegenüberliegenden knochenkontaktierenden Fläche.

Bei den erfindungsgemäßen Implantaten enden die Kanäle vorzugsweise nicht auf der Höhe des äußeren Mantels, sondern gehen bis maximal zu 10 mm über dessen Höhe hinaus.

Die Ausgestaltung der Kanäle ist erfindungswesentlich und das erfindungsgemäße Kanalnetzwerk folgt einer Symmetrie. Es sei angemerkt, dass ein zufällig entstandenes Kanalnetzwerk, wie es beispielweise ohne Symmetrie in porösen Strukturen oder Schwämmen vorliegt, die erfindungsgemäße Aufgabe nicht löst. Gleiches gilt für Kanäle, welche sprunghaft ihre Richtung und ihren Durchmesser ändern oder sich aufgrund mehrlagiger Systeme zufällig und/oder in willkürlicher Abfolge und/oder Form ergeben. Bei derartigen Systemen zeigt sich, dass der Blutfluss nur in Teilbereichen verstärkt wird und sich nur in Teilbereichen oder punktförmig Knochenzellbildungen zeigen, ein Durchwachsen des gesamten Implantats mit Knochenzellen teilweise nicht oder insgesamt nur verlangsamt stattfindet.

Erfindungsgemäß verlaufen die Kanäle im wesentlichen parallel zueinander und sind geradlinig, d.h. die Kanäle weisen keine Biegungen, Kurven, Krümmungen oder ähnliches auf, sonder verlaufen von ihrer Öffnung auf einer äußeren Fläche des Implantats im wesentlichen parallel in das Implantat oder eines Teils des Implantats hinein und enden im Inneren des Implantats oder verlaufen vorzugsweise durch das Implantat bis zur gegenüberliegenden äußeren Fläche des Implantats. Zudem ändern die Kanäle vorzugsweise weder kontinuierlich noch sprunghaft oder stufenweise ihren Radius oder Durchmesser, ungeachtet, ob es sich um runde, ovale oder mehreckige Kanäle handelt.

Unter dem Begriff "im wesentlichen parallel" soll verstanden werden, dass gewisse Herstellungstoleranzen vorliegen können und abgesehen von diesen Toleranzen die Kanäle parallel zueinander verlaufen.

Ferner verändern die Kanäle in ihrem Verlauf nicht ihren Durchmesser, d.h. ebenfalls abgesehen von Herstellungstoleranzen, haben die Kanäle im wesentlichen von ihrem Anfang bis zu ihrem Ende denselben Durchmesser.

Ferner ist auch nicht zwingend, dass alle Kanäle auf der oder einer knochenkontaktierenden Oberfläche beginnen, d.h. unmittelbar mit dem Knochen in Berührung stehen. Bis zu 30% vorzugsweise bis zu 20% aller Kanäle kann auch in einem Bereich des Implantats beginnen, der nicht direkt mit dem Knochen in Berührung steht, d.h. vorzugsweise beginnen diese Kanäle seitlich der knochenkontaktierenden Oberfläche.

Hingegen ist nicht erforderlich, dass die Kanäle auch an einer knochenkontaktierenden Oberfläche enden, was sowieso nur bei einteiligen knochenverbindenden oder knochenüberbrückenden Implantaten der Fall wäre. Bis zu 100% aller Kanäle können auch einer nicht einen Knochen kontaktierenden Oberfläche enden, wobei es jedoch auch möglich ist, dass bis zu 100% der Kanäle auf einer gegenüberliegenden anderen knochenkontaktierenden Oberfläche enden, was aus herstellungstechnischen Gründen für die einteiligen erfindungsgemäßen Cages bevorzugt ist.

Zudem ist bevorzugt, wenn abgesehen von dem massiven Außenmantel des erfindungsgemäßen Implantats, pro cm² knochenkontaktierender Oberfläche mindestens 50 Kanäle, vorzugsweise mindestens 100 Kanäle und insbesondere bevorzugt mindestens 150 Kanäle anfangen. Die erfindungsgemäße Kanalstruktur weist zwischen 20 und 1000 Kanäle pro cm² auf, vorzugsweise zwischen 50 und 750, weiter bevorzugt zwischen 100 und 500, noch weiter bevorzugt zwischen 125 und 350 und insbesondere bevorzugt zischen 150 und 250 Kanäle pro cm² auf.

Zudem ist erfindungswesentlich, dass die einzelnen Kanäle untereinander verbunden sind. Die Kanäle werden durch Öffnungen miteinander verbunden, wobei jeder Kanal zumindest eine Öffnung zu einem angrenzenden Kanal hat. Ferner ist bevorzugt, wenn die äußeren Kanäle, d.h. diejenigen Kanäle, welche die äußere Reihe der gesamten kanalartigen Struktur bilden und an den massiven Außenmantel grenzen, zumindest eine Öffnung zu einem benachbarten Kanal besitzen und die Kanäle, welche im Inneren der kanalartigen Struktur liegen zumindest eine Öffnungen zu zwei verschiedenen angrenzenden Kanälen besitzen, d.h. also absolut mindestens zwei Öffnungen aufweisen.

Zudem ist bevorzugt, wenn die Öffnungen so angeordnet werden, dass alle Kanäle miteinander verbunden sind, d.h. theoretisch über eine Öffnung eines Kanals das gesamte kanalartige System mit Flüssigkeit wie z.B. Blut gefüllt werden könnte. Man erzeugt also vorzugsweise eine dreidimensionale Interkonnektivität der gesamten Struktur.

Die Öffnungen oder auch Verbindungskanäle genannt, können beliebig ausgestaltet sein und in Form von Löchern oder Schnitten vorliegen, rund, kreisförmig, punktförmig, zylinderförmig, oval, eckig, keilförmig oder eine andere beliebige Ausgestaltung aufweisen.

Es ist darüber hinaus bevorzugt, dass auch die Öffnungen zwischen den Kanälen einem Muster, d.h. einer Symmetrie oder einer wiederkehrenden Anordnung folgen. Somit ist bevorzugt, dass die Öffnungen zwischen den Kanälen entweder entlang der Längsachse der Kanäle verlaufen und die Öffnungen eine maximale Länge aufweisen können, welche der Länge der miteinander verbundenen Kanäle entspricht. Bei dieser Art von Öffnungen, welche entlang der Längsachse der Kanäle verlaufen, handelt es sich vorzugsweise um Einschnitte vorzugsweise keilförmige Einschnitte in die Kanalwandungen bzw. Kanalwände.

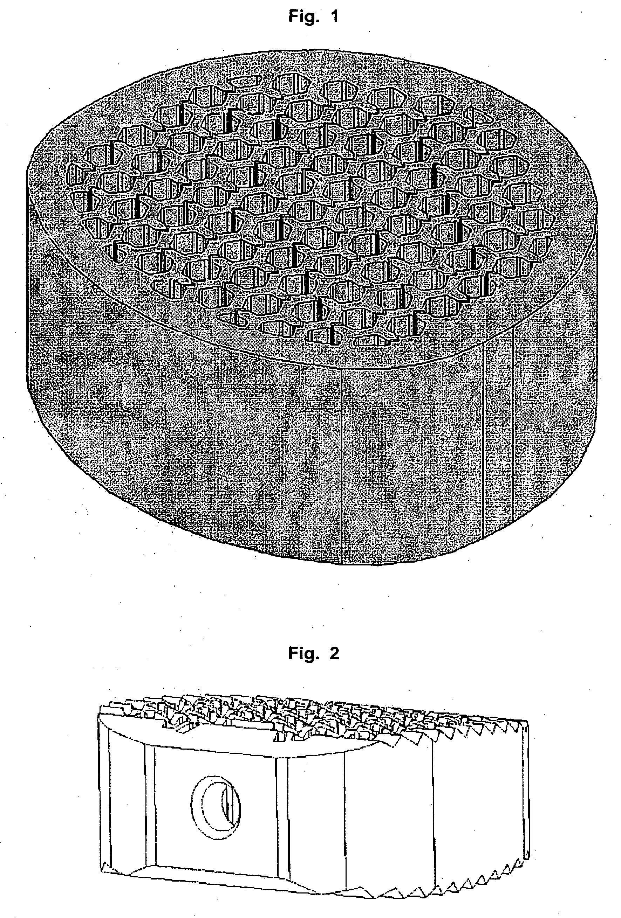

Eine andere Art von Öffnungen ist vorzugsweise rund oder oval ausgestaltet und verläuft senkrecht zur Längsachse der Kanäle. Die Längsachse der Kanäle verläuft in Richtung der Längsachse der Wirbelsäule. Diese Öffnungen werben beispielsweise per Laser in das Implantat geschnitten und verlaufen durch die Außenwand des Implantats in Richtung der gegenüberliegenden Fläche und verbinden so die auf dieser Geraden liegenden Kanäle untereinander. Um die oben beschriebene Stabilität zu gewährleisten, können die Öffnungen oder Verbindungskanäle auch im Innern des Cages aufhören, ohne den gegenüberliegenden Außenmantel zu durchdringen. Somit ist bevorzugt, dass diese Verbindungskanäle durch den Außenmantel gehen und vor der Innenfläche des gegenüberliegenden Außenmantels enden. Bevorzugt sind jedoch Öffnungen oder Einschnitte, welche entlang der Mittelachse der Kanäle verlaufen und als Schnitt oder Keilschnitt die Wand eines Kanals entlang seiner Gesamtlänge durchtrennen. Bei derartigen Längsschnitten entlang der Kanalwand Werden diese selbstverständlich so angeordnet, dass durch mehrere Schnitte in benachbarten Kanalwänden keine Teilstücke von Kanalwänden aus der Gesamtstruktur herausgetrennt werden. Betrachtet man hierzu Figur 2 mit den sechseckigen Kanälen und den keilförmigen Verbindungskanälen oder Einschnitten, so könnte man die Kanalwände in laterale Wandbereicht und anteriore-posteriore Wandbereicht unterteilen. In Figur 2 sind beispielsweise nur die lateralen Wandbereiche eingeschnitten, so dass alle Kanalwände mindestens an zwei Stellen mit dem massiven Außenmantel verbunden sind und kein Wandsegment auch nicht ein Segment aus mehreren Kanalwandbereichen von mehreren Kanälen aus der Gesamtkanalstruktur herausgeschnitten oder herauslösbar ist.

Der Durchmesser oder die Dicke der Öffnungen liegt im Bereich von 0,1 µM bis 1000 µm, vorzugsweise im Bereich 1 µM bis 500 µM, weiter bevorzugt 10 µM bis 200 µM, noch weiter bevorzugt im Bereich von 30 µM bis 100 µM und insbesondere bevorzugt im Bereich von 50 bis 80 µm.

Ferner können sich die Öffnungen entlang der Längsachse der Kanäle erstrecken, was als durchgehend bezeichnet wird und sogar von einer knochenkontaktierenden Oberfläche bis zur gegenüberliegenden knochenkontaktierenden Oberfläche verlaufen und somit die Länge der Kanäle selbst besitzen.

Die Öffnungen können zudem in Form von Bohrungen senkrecht zur Längsachse der Kanäle durch das Implantat verlaufen oder als nach bestimmten Abständen wiederkehrende Öffnungen in den Kanalwänden die Kanäle miteinander verbinden.

Die Ausgestaltung der Kanäle an sich ist nicht erfindungswesentlich, sondern deren Vorhandensein. Es versteht sich für einen Fachmann, dass zu viele Öffnungen die Stabilität des Implantats beeinträchtigen können, so dass ein Fachmann Anzahl, Größe und Lage der Öffnungen nach der Art des Implantats versteht zu bestimmen.

Ferner sollte der Durchmesser oder die Dicke der Öffnungen kleiner sein, als der Durchmesser oder die Dicke der Kanäle und vorzugsweise weniger als ein Zehntel der Dicke der Kanäle betragen.

Grundsätzlich könnte man die Öffnungen auch als senkrecht zur Längsachse der Kanäle verlaufende Verbindungskanäle bezeichnen. Die erfindungsgemäße Kanalstruktur besteht ferner vorzugsweise aus im wesentlichen parallel verlaufenden Kanälen, welche ferner vorzugsweise parallel zu den Verbindungskanälen verlaufen.

Es versteht sich zudem von selbst, dass nicht das gesamte Implantat die erfindungsgemäße Kanalstruktur aufweisen muss, sondern nur die Bereiche eines Implantats, welche mit dem Knochen in Berührung kommen oder insbesondere in den Knochen eingelassen werden. Dennoch ist bei den erfindungsgemäßen Cages oder Zwischenwirbelimplantaten bevorzugt, wenn die Kanalstruktur sich im Innern des Implantats von der Unterseite des aufliegenden Wirbelkörpers bis zur Oberseite des unterliegenden Wirbelkörpers erstreckt. Das Inneren des Implantats wird durch den Außenmantel definiert.

Bei den Ausführungsformen von Cages haben sich insbesondere durchgehende im wesentlichen parallele Kanäle als vorteilhaft erwiesen, welche durch keilförmige Längsschnitte entlang der Längsachse der Kanäle miteinander verbunden sind, so wie in Figur 2, 3 und 4 dargestellt.

Ferner ist bei den erfindungsgemäßen Implantaten bevorzugt, wenn sich die Wabenstruktur, d.h. die innere Kanalstruktur ein wenig über die im wesentlichen ebene knochenkontaktierende Fläche erhebt. Ragt insbesondere bei solchen Implantaten die Wabenstruktur über einen massiven Rand hinaus, ergeben sich die Vorteile einer hohen Oberflächenreibung und damit einer sehr guten Verankerung und gleichzeitig aufgrund der geringen Dicke der Wabenwandungen die Möglichkeit der mechanischen Bewegung derselben, was den Wachstumsreiz des Knochens fördert.

Die Wände zwischen den einzelnen Kanälen, d.h. die Wabenwandungen oder Kanalwandungen haben eine Dicke von 1 µm bis 3.000 µm, vorzugsweise 5 µm bis 1.000 µm, weiter bevorzugt 10 µm bis 500 µm und insbesondere bevorzugt 50 µm bis 250 µm.

Zudem ist bevorzugt, dass die Öffnungen in der inneren Kanalstruktur derart angeordnet sind, dass die gesamte Struktur Mikrobewegungen vorzugsweise Friktionsbewegungen zulässt. Derartige Bewegungen ermöglicht z.B. eine Struktur wie in Figur 2 gezeigt, worin die einzelnen Kanäle durch keilförmige Längsschnitte in den lateralen Wandbereichen entlang der Längsachse der Kanäle miteinander verbunden sind. Dabei können die einzelnen Kanalwandungen entsprechend der Dicke der keilförmigen Öffnungen gegeneinander verschoben werden, so dass Mikrobewegungen möglich werden.

Kombiniert man ferner diese Art der Ausgestaltung der Öffnungen zwischen den Kanälen mit der Ausgestaltung, dass die innere kanalartige Struktur sich wie eine Insel bis zu mehreren Millimetern über die im wesentliche ebene knochenkontaktierende Fläche erhebt, d.h. konvex in Richtung des kontaktierten Wirbelkörpers ausgestaltet ist, so regt dieser bis zu 10 mm erhobene Teilbereich der Wabenstruktur das Knochenwachstum besonders gut an, da sich dieser erhobene Teil in den Knochen leicht eingräbt und durch seine Eigenschaft Mikrobewegungen zuzulassen, den Knochenbewegungen folgen kann und ferner ganz besonders gut das Knochenwachstum durch eine leichte aber kontinuierliche Stimulierung fördert.

Das Knochenwachstum anregende Oberflächenausgestaltungen von Implantaten sind noch immer ein Gegenstand der Forschung, ohne bisher zu einem befriedigenden Ergebnis geführt zu haben. Die vorher beschriebene erhobene Wabenstruktur mit der Fähigkeit zu Mikrobewegungen, insbesondere Mikrofriktionsbewegungen scheint die lange gesuchte Lösung darzustellen, das Knochenwachstum in optimaler Weise anzuregen und zu einer raschen Durchbauung des gesamten Implantats zu führen.

Diese erfindungsgemäße Kanalstruktur für die knochenkontaktierenden, knochenverbindenden oder knochenüberbrückenden Implantate hat sich überraschend als sehr vorteilhaft in Bezug auf ein Einwachsen von Knochengewebe und ein festes Verwachsen mit dem kontaktierten Knochen erwiesen.

Zudem vereint die erfindungsgemäße Wabenstruktur die Eigenschaften guter mechanischer Stabilität bei gleichzeitig erhaltenem optimalem Befüllvolumen, so dass eine rasche und stabile Durchwachsung des Implantats mit Knochen stattfindet.

Knochengewebe umfasst in der Regel drei Zellformen, die Osteoblasten, die Osteozyten und die Osteoklasten, wobei der ausgebildete Knochen zudem eine Knochendeckschicht aus Knochendeckzellen besitzt. Das Vorhandensein von Blut ist essentiell erforderlich für eine optimale Knochenbildung. Bei der Bildung von Knochen arbeiten die Osteoblasten, Osteozyten und Osteoklasten zusammen. Osteoblasten sind knochenproduzierende Zellen und für den Aufbau und Erhalt des Knochens verantwortlich. Nicht aktive Osteoblasten an der Knochenoberfläche werden Knochendeckzellen genannt. Osteozyten sind ehemaligen Osteoblasten, die durch den Verknöcherungsprozess im Knochengewebe eingebaut sind. Sie sorgen für den Erhalt des Knochens indem sie den Knochenabbau dem Aufbau anpassen. Osteoklasten sind für den Abbau des Knochens verantwortlich. Durch sie kann die Dicke des Knochens bestimmt und Calcium und Phosphat aus dem Knochen freigesetzt werden. Die Osteoblasten sind Zellen, die für die Knochenbildung verantwortlich sind. Sie entwickeln sich aus undifferenzierten Mesenchymzellen, embryonalen Bindegewebszellen. Sie lagern sich an Knochen hautschichtartig an und bilden indirekt die Grundlage für neue Knochensubstanz, die Knochenmatrix, indem sie vor allem Calciumphosphate und -carbonate in den interstitiellen Raum ausscheiden. Bei diesem Prozess verändern sie sich zu einem Gerüst aus nicht mehr teilungsfähigen Osteozyten, das langsam mineralisiert und mit Calcium aufgefüllt wird.

Die erfindungsgemäße Kanalstruktur scheint das Einfließen von Blut und damit Osteoblasten zu begünstigen, welche zügig die Kanalräume ausfüllen und zu einem deutlich besseren Verwachsen des Implantats mit dem Knochen führen, als herkömmliche Implantate dazu in der Lage sind.

Zudem besitzen die erfindungsgemäß ausgestalteten Implantate gegenüber beispielsweise porösen Strukturen und Schwämmen den Vorteil, dass sie wenig deformierbar und formstabil sind, eine definierte Gestalt und Oberfläche besitzen und durch herkömmliche Implantationswerkzeuge gehandhabt und implantiert werden können, ohne Gefahr zu laufen, das Implantat oder die kanalartige Struktur im Implantat zu zustören oder zu beschädigen.

Um das Anhaften von Knochenzellen noch weiter zu begünstigen, können die Innenflächen der Kanäle noch strukturiert werden, wozu beispielsweise eine mechanische, chemische oder physikalische Aufrauung zählt. Um das Wachstum von Bakterien oder anderen Keimen auf der Implantatsoberfläche zu unterdrücken, kann diese mit Antibiotika und die Außenfläche des Außenmantels beispielsweise mit einer wirkstofffreisetzenden Beschichtung versehen werden, worin Wirkstoffe wie beispielsweise Antibiotika eingelagert und kontinuierlich freigesetzt werden können.

### Figurenbeschreibung

- Figur 1: zeigt eine perspektivische Ansicht eines erfindungsgemäßen knochenverbindenden Bandscheibenimplantats wie z.B. eines Cages. Beispielhaft ist das Implantat mit ovaler Form dargestellt, welche jedoch nicht zwingend ist. Die Außenwand des Implantats ist massiv und das Implantat besteht aus einem physiologisch verträglichen Material, insbesondere einem Metall oder einer Metalllegierung. Im inneren Bereich des Implantats ist die wabenförmige Struktur aus durchgehenden Kanälen zu erkennen, welche untereinander durch Öffnungen miteinander verbunden sind,
- Figur 2: zeigt das erfindungsgemäße Wirbelzwischenimplantat von einer seitlichen Ansicht.
- Figur 3: zeigt eine Aufsicht des Implantats entlang der Längsachse der Kanäle. Die Seitenwände der Kanäle sind als Wabenmuster zu erkennen und die Öffnungen zwischen den Kanälen sind als keilförmige Einschnitte sichtbar. Zudem ist zu erkennen, dass die Kanäle durchgehend sind, d.h. von der Oberseite des Implantats zur Unterseite durchlaufen.
- Figur 4: zeigt ein erfindungsgemäßes Wirbelzwischenimplantat von einer perspektivischen Ansicht. Es ist gut die gezahnte Oberseite und auch die gezahnte Unterseite des Implantats erkennbar, welche zur Stabilisierung der Lage des Implantats beiträgt.

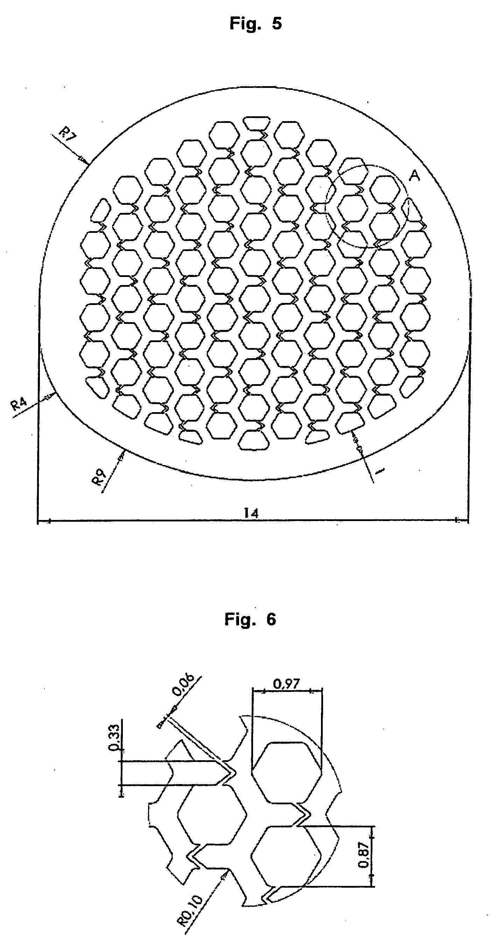

- Figur 5: zeigt nochmals die erfindungsgemäße Wabenstruktur wie in Figur 3 und markiert einen Ausschnitt, der in Figur 6 vergrößert dargestellt wird.
- Figur 6: zeigt den in Figur 5 markierten Ausschnitt. Die sechseckige Struktur der Kanäle und die keilförmigen Öffnungen zwischen den Kanälen sowie die Wandungen der Kanäle sind vergrößert dargestellt.

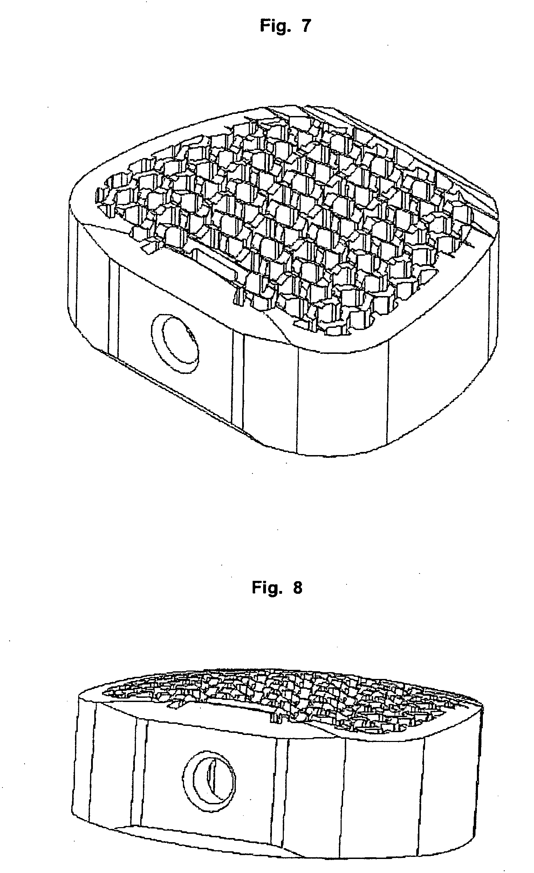

- Figur 7: zeigt eine Ausführungsform eines erfindungsgemäßen Cages mit einer konvexen und gezahnten Oberseite und einer konvexen und gezahnten Unterseite (nicht sichtbar) bzw. einer konvex erhobenen inneren wabenartigen Struktur.
- Figur 8: zeigt eine weitere Ansicht des erfindungsgemäßen Cages gemäß Figur 7, wobei die konvex ausgestaltete Oberseite und die konvex ausgestaltete Unterseite gut zu erkennen sind.
- Figur 9: zeigt eine Frontansicht des erfindungsgemäßen Cages gemäß Figur 7 mit deutlich erkennbarer konvexer Oberseite und Unterseite. Die mittig gezeigte runde Aussparung dient zur Aufnahme eines Implantationswerkzeugs.
- Figur 10: zeigt eine seitliche Ansicht eines erfindungsgemäßen Wirbelzwischenimplantals mit einer gezahnten Oberseite und gezahnten Unterseite. Die Zahnung befindet sich in der wabenförmigen Struktur als auch im Außenmantel und dient zur Stabilisierung der Lage des Implantats zwischen den Wirbelkörpern nach der Implantation.

### Ausführungsbeispiele

Bevorzugte Ausführungsformen der erfindungsgemäßen Vorrichtung werden nun anhand der Beispiele diskutiert, wobei zu berücksichtigen ist, dass die diskutierten Beispiele vorteilhafte Ausgestaltungen der Erfindung wiedergeben, jedoch den Schutzumfang nicht auf diese Ausführungsformen beschränkten.

### Beispiel 1: Cage

Beispiel 1 betrifft einen Cage, insbesondere einen cervicalen Cage mit einem Längsdurchmesser von 14 mm und einem Querdurchmesser von 12 mm und einer Höhe von 8 mm. Der Cage ist annähernd oval und als Längsdurchmesser wird der größtmögliche Durchmesser und als Querdurchmesser der kleinstmögliche Durchmesser verstanden.

Der Cage besteht aus Titan mit einem massiven mindestens 1,1 mm dicken Außenmantel und einer oberen und unteren ebenen Fläche für den Kontakt mit dem jeweiligen Wirbelkörper.

Im Inneren des Cages ist eine wabenförmige Struktur aus Kanälen mit sechseckigen Wandungen ausgebildet. Die Kanäle erstrecken sich geradlinig von der oberen den Knochen kontaktierenden Oberfläche zur unteren gegenüberliegenden den anderen Wirbelkörper kontaktierenden ebenen Oberfläche. Pro cm² knochenkontaktierender Oberfläche sind ca. 34 - 42 Kanäle vorhanden.

Die Kanäle haben einen Durchmesser angegeben als Abstand zweier gegenüberliegender paralleler Wandflächen von 870 - 970 µm.

Die Kanäle sind zudem über Aussparungen in den Kanalwänden miteinander verbunden. Die Aussparungen weisen eine keilförmige Struktur auf, so wie in Figur 2 gezeigt, so dass die Kanalwände lateral nur um die Dicke der Aussparung gegeneinander verschoben werden können, was zu einer Erhöhung der Stabilität des Implantats beiträgt. Die Aussparung weist eine Dicke von 60 µm auf.

### Beispiel 2: Cage

Beispiel 2 betrifft einen Cage, insbesondere einen cervicalen Cage mit einem Längsdurchmesser von 16 mm und einem Querdurchmesser von 13 mm und einer Höhe von 9 mm. Der Cage ist annähernd oval und als Längsdurchmesser wird der größtmögliche Durchmesser und als Querdurchmesser der kleinstmögliche Durchmesser verstanden.

Der Cage besteht aus Zirkonium mit einem massiven 1,2 mm dicken Außenmantel und einer oberen und unteren Fläche für den Kontakt mit dem jeweiligen Wirbelkörper. Der obere Rand des Außenmantels ist eben und dient zur Auflage des oberen Wirbelkörpers. Die innere Kanalstruktur erhebt sich ausgehend vom Rand des Außenmantels in konvexer Form bis zu 4 mm über den Rand des Außenmantels hinaus, so dass sich die Kanalstruktur in der Mitte des Cages bis zu 4 mm in die Unterseite des aufliegenden Wirbelkörpers eindrücken kann. Auf der gegenüberliegenden Seite des Cages erstreckt sich ebenfalls die innere Waben- bzw. Kanalstruktur wie ein Teil einer Kugeloberfläche in konvexer Form in Richtung der oberen Fläche des unterliegenden Wirbelkörpers und gräbt sich ebenfalls im mittleren Bereich bis zu 4 mm und in den Randbereichen entsprechend weniger in den unteren Wirbelkörper ein.

Im Inneren des Cages ist eine wabenförmige Struktur aus runden Kanälen ausgebildet. Die Kanäle erstrecken sich geradlinig von der oberen den oberen Wirbelkörper kontaktierenden Oberfläche zur unteren den gegenüberliegenden anderen Wirbelkörper kontaktierenden Oberfläche. Pro cm² knochenkontaktierender Oberfläche sind ca. 40 Kanäle vorhanden.

Die Kanäle haben einen Durchmesser von 850 µm und die Wandstärke der Kanalwände beträgt 350 µm.

Die Kanäle sind zudem über Aussparungen in den Kanalwänden miteinander verbunden, welche in Form von Längsschnitten ausgestaltet sind. Diese Längsschnitte durchtrennen die Kanalwand in ihrer gesamten Länge. Die Längsschnitte durchtrennen die Kanalwand jedoch nicht auf dem kürzesten Weg, der 350 µm beträgt, sondern durchtrennen die Kanalwand schräg auf einer Strecke von ca. 370 µm in z.B. Ost-West-Richtung. Die gegenüberliegende Seite des Kanals wir wiederum mittels Längsschnitt schräg auf einer Strecke von ca. 370 µm durchtrennt, nun aber in West-Ost-Richtung. Die Dicke des Längsschnitts, d.h. des Verbindungskanals beträgt 50 µm.

Eine derartige Wabenstruktur lässt Mikrobewegungen zu und gräbt sich in aufliegenden und unterliegenden Wirbelkörper bis jeweils maximal zu 4 mm ein und vermag das Knochenwachstum sehr start anzuregen, so dass eine schnelle und gute Durchwachsung des Implantats mit neugebildetem Knochen stattfindet.

### Beispiel 3: Cage

Beispiel 3 betrifft einen Cage, insbesondere einen thorakalen Cage mit einem Längsdurchmesser von 10 mm und einem Querdurchmesser von 8,8 mm und einer Höhe von 6,5 mm. Der Cage ist annähernd oval und als Längsdurchmesser wird der größtmögliche Durchmesser und als Querdurchmesser der kleinstmögliche Durchmesser verstanden.

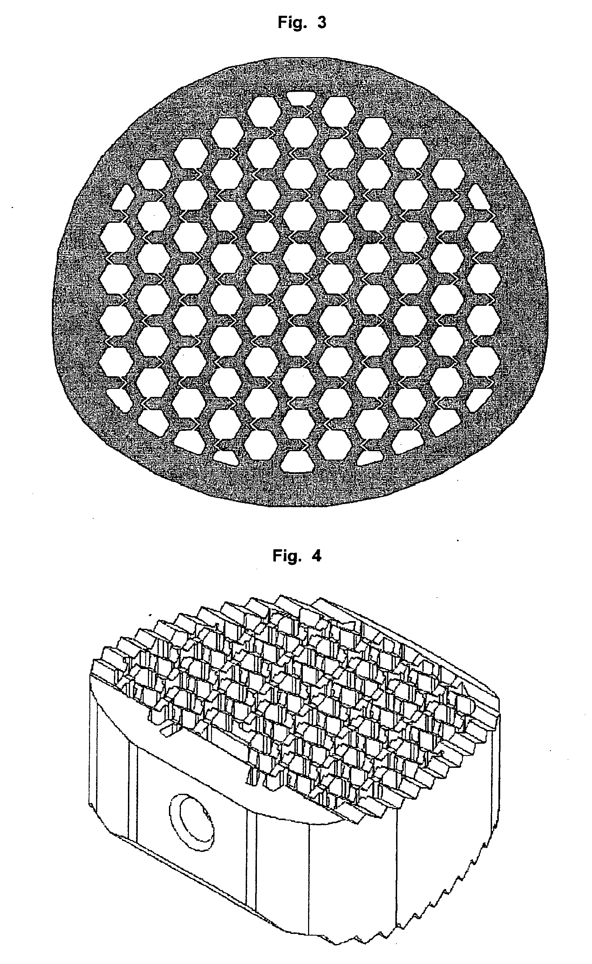

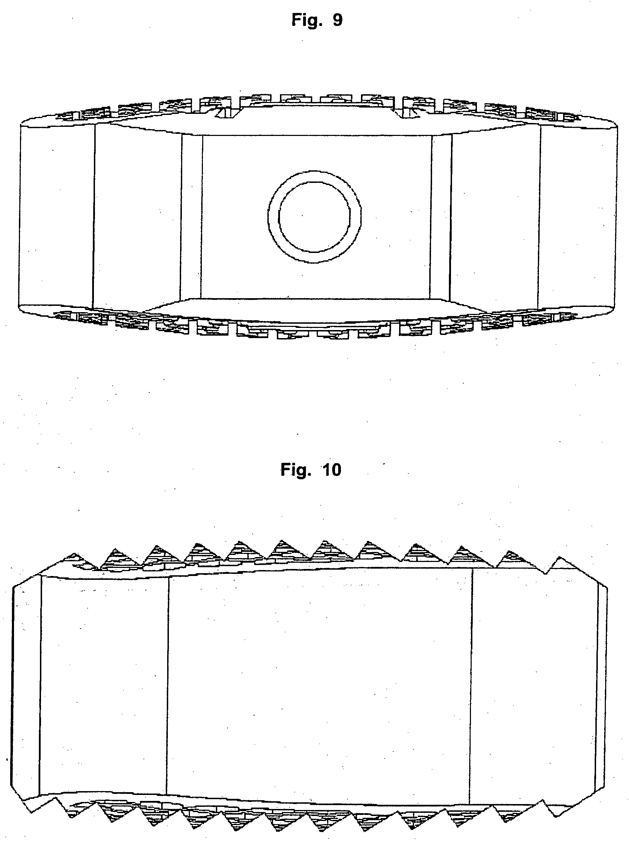

Der Cage besteht aus in der Medizintechnik eingesetztem PEEK mit einem massiven mindestens 0,9 mm dicken Außenmantel und einer oberen und unteren ebenen Fläche für den Kontakt mit dem jeweiligen Wirbelkörper, wobei die Oberseite als auch die Unterseite des Cages gezackt oder gezahnt mit einer Zahnhöhe von ca. 0,5 mm. Derartig ausgestaltete Ober- und Unterseiten sind beispielsweise in Figur 4 und Figur 10 gezeigt.

Im Inneren des Cages ist eine kanalförmige Struktur aus Kanälen mit viereckigen Wandungen ausgebildet. Die Kanäle erstrecken sich geradlinig von der oberen den Knochen kontaktierenden Oberfläche zur unteren gegenüberliegenden den anderen Wirbelkörper kontaktierenden ebenen Oberfläche. Pro cm² knochenkontaktierender Oberfläche sind ca. 30 - 33 Kanäle vorhanden.

Die Kanäle haben einen Durchmesser angegeben als Abstand zweier gegenüberliegender paralleler Wandflächen von ca. 800 µm.

Die Kanäle sind zudem über Aussparungen oder Einschnitten in den Kanalwänden miteinander verbunden. Die Aussparungen oder Einschnitte weisen eine geradlinige Struktur auf und durchtrennen die Kanalwände auf dem kürzesten Weg, wobei nur zueinander parallele Kanalwände durchtrennt werden, damit keine Kanalwandbestandteile aus der kanalartigen Struktur herausgelöst werden. Die Aussparungen oder Einschnitte weisen eine Dicke von 30 µm auf.

### Beispiel 4: Cage

Der Cage gemäß Beispiel 4 besteht aus Titan und hat die Abmessungen wie der Cage gemäß Beispiel 1 und weist darüber hinaus eine gezahnte Oberseite und eine gezahnte Unterseite auf mit einer maximalen Zahnhöhe von 0,75 mm.

Des weiteren sind die Aussparungen in den Kanalwandungen nicht keilförmige und erstrecken sich auch nicht über die gesamte Länge der Kanalwand. Die Aussparungen sind hingegen als ovale Langlöcher in den Kanalwänden ausgestaltet mit einem Querdurchmesser von 7 µm und einem Längsdurchmesser von 20 µm.

## Patentansprüche

1. Zwischenwirbelimplantat, wobei das Implantat zwei Oberflächen zur Kontaktierung von zwei Wirbelkörpern, einen äußeren Mantel und eine innere Struktur aufweist und wobei die innere Struktur aus einer Vielzahl von Kanälen gebildet wird und die Kanäle jeweils eine Querschnittsfläche von 8.000 µm² bis 7.000.000 µm² besitzen und die Kanäle parallel zueinander entlang der Längsachse der Wirbelsäule verlaufen und die Kanäle über Öffnungen miteinander verbunden sind, **dadurch gekennzeichnet, dass** jeder Kanal durch mindestens zwei Öffnungen mit benachbarten Kanälen verbunden ist und sich die Öffnungen durchgehend von einer knochenkontaktierenden Oberfläche zur gegenüberliegenden Oberfläche in Form von Schnitten erstrecken.

2. Zwischenwirbellmplantat gemäß Anspruch 1, wobei die Kanäle einen Durchmesser von 100 µm bis 3000 µm besitzen.

3. Zwischenwirbelimplantat gemäß Anspruch 1 oder 2, wobei die den Knochen kontaktierende Oberfläche der inneren Struktur konvex ist.

4. Zwischenwirbelimplantat gemäß eines der vorhergehenden Ansprüche, wobei die Kanäle sich durchgehend von einer knochenkontaktierenden Oberfläche zur gegenüberliegenden Oberfläche erstrecken.

5. Zwischenwirbelimplantat gemäß eines der vorhergehenden Ansprüche, wobei das Implantat pro cm² knochenkontaktierender Oberfläche mindestens 100 Kanäle aufweist.

6. Zwischenwirbelimplantat gemäß eines der vorhergehenden Ansprüche, wobei die Öffnungen punktförmig, kreisförmig, zylinderförmig, oval oder keilförmige sind.

7. Zwischenwirbelimplantat gemäß Anspruch 1, wobei die Öffnungen sich entweder nur In den lateralen Bereichen oder nur in den anterioren-posterioren Bereichen der Kanalwände befinden.

8. Zwischenwirbelimplantat gemäß eines der vorhergehenden Ansprüche, wobei Kanäle rund, oval, dreieckig, viereckig, fünfeckig oder sechseckig ausgestaltet sind.

9. Zwischenwirbelimplantat gemäß eines der vorhergehenden Ansprüche, wobei Kanäle ihren Radius oder Durchmesser während des Verlaufs nicht ändern.

10. Zwischenwirbelimplantat gemäß eines der vorhergehenden Ansprüche, wobei die Öffnungen in Form von Bohrungen senkrecht zur Längsachse der Kanäle durch das Implantat verlaufen.

11. Zwischenwirbelimplantat gemäß eines der vorhergehenden Ansprüche, wobei das Implantat aus einem Metall oder einer Metalllegierung oder aus PEEK besteht.

12. Zwischenwirbelimplantat gemäß eines der vorhergehenden Ansprüche, wobei die innere Struktur des Implantats eine Mikrobewegung aufgrund von keilförmigen oder schrägen Öffnungen in Form von Längsschnitten entlang der Kanalwand zulässt.

13. Zwischenwirbelimplantat gemäß eines der vorhergehenden Ansprüche, wobei das Implantat ausgewählt wird aus der Gruppe umfassend cervicale Cages, thorakale Cages, lumbale Cages, künstliche Bandscheiben und Implantate für die Fusion von Wirbeln.

## Claims

1. Intervertebral implant, wherein the implant has two surfaces for contacting two vertebral bodies, an outer sheath and an inner structure and wherein the inner structure is formed by a plurality of channels and the channels each have a cross-sectional area of 8,000 µm² to 7,000,000 µm² and the channels extend parallel to one another along the longitudinal axis of the spinal column and the channels are connected by openings to each other, **characterized in that** each channel is connected with at least two openings with the adjacent channels and the openings extend continuously from one bone-contacting surface to the opposite surface in the form of cuts.

2. Intervertebral implant according to claim 1, wherein the channels have a diameter of 100 µm to 3,000 µm.

3. Intervertebral implant according to any one of claim 1 or 2, wherein the bone-contacting surface of the inner structure is convex.

4. Intervertebral implant according to any of the preceding claims, wherein the channels extend continuously from one bone-contacting surface to the opposite surface.

5. Intervertebral implant according to any of the preceding claims, wherein the implant has at least 100 channels per cm² of bone-contacting surface.

6. Intervertebral implant according to any of the preceding claims, wherein the openings are point-shaped, punctiform, circular, cylindrical, oval or wedge-shaped.

7. Intervertebral implant according to claim 1, wherein the openings are located either only in the lateral areas or only in the anterior-posterior areas of the channel walls.

8. Intervertebral implant according to any of the preceding claims, wherein the channels are shaped round, oval, triangular, square, pentagonal or hexagonal.

9. Intervertebral implant according to any of the preceding claims, wherein the channels do not change their radius or diameter along the pathway.

10. Intervertebral implant according to any of the preceding claims, wherein the openings in the form of drill-holes run vertical to the longitudinal axis of the channels through the implant.

11. Intervertebral implant according to any of the preceding claims, wherein the implant consists of metal, or a metal alloy, or PEEK.

12. Intervertebral implant according to any of the preceding claims, wherein the inner structure of the implant permits micro-movements due to wedge-shaped or oblique openings in the form of longitudinal cuts along the channel wall,

13. Intervertebral implant according to any of the preceding claims, wherein the implant is selected from the group comprising cervical cages, thoracic cages, lumbar cages, artificial intervertebral discs and implants for the fusion of vertebrae.

## Revendications

1. Un implant intervertébral, où l'implant a deux surfaces pour contacter deux corps vertébraux, une enveloppe extérieure et une structure intérieure et où la structure intérieure est formée par une pluralité de canaux et chacun des canaux a une surface de section transversale de 8000 µm² à 7000000 µm² et les canaux s'étendent parallèlement les uns aux autres le long de l'axe longitudinale de la colonne vertébrale et les canaux sont connectés les uns aux autres par des ouvertures, **caractérisé en ce que** chaque canal est connecté aux canaux voisins par au moins deux ouvertures et les ouvertures s'étendent continuellement sous forme de coupures d'une surface contactant l'os à la surface opposée.

2. L'implant intervertébral selon revendication 1, dans lequel les canaux possèdent un diamètre de 100 µm à 3000 µm.

3. L'implant intervertébral selon revendication 1 ou 2, où la surface contactant l'os de la structure intérieure est convexe.

4. L'implant intervertébral selon une quelconque des revendications précédentes, dans lequel les canaux s'étendent continuellement d'une surface contactant l'os à la surface opposée.

5. L'implant intervertébral selon une quelconque des revendications précédentes, où l'implant a au moins 100 canaux par cm² de surface contactant l'os.

6. L'implant intervertébral selon une quelconque des revendications précédentes, où les ouvertures sont ponctuelles, circulaires, cylindriques, ovales ou en forme de coin.

7. L'implant intervertébral selon revendication 1, où les ouvertures sont situées soit seulement dans les zones latérales ou seulement dans les zones antérieur-postérieur des parois du canal.

8. L'implant intervertébral selon une quelconque des revendications précédentes, où les canaux ont une forme ronde, ovale, triangulaire, carrée, pentagonale ou hexagonale.

9. L'implant intervertébral selon une quelconque des revendications précédentes, dans lequel les canaux ne changent pas de rayon ou de diamètre au long du chemin.

10. L'implant intervertébral selon une quelconque des revendications précédentes, dans lequel les ouvertures, sous forme de trous de perçage, s'étnedent à travers l'implant perpendiculairement à l'axe longitudinal des canaux.

11. L'implant intervertébral selon une quelconque des revendications précédentes, où l'implant est constitué d'un métal, d'un alliage de métal ou de PEEK.

12. L'implant intervertébral selon une quelconque des revendications précédentes, dans lequel la structure interne de l'implant permet un micro - mouvement grâce aux ouvertures en forme de coin ou obliques, qui sont sous forme de coupures longitudinales au long de la paroi du canal.

13. L'implant intervertébral selon une quelconque des revendications précédentes, où l'implant est sélectionné à partir du groupe comprenant des cages cervicales, des cages thoraciques, des cages lombaires, des disques intervertébraux artificiels et des implants pour la fusion des vertèbres.
